(19) Europäisches Patentamt / European Patent Office / Office européen des brevets

(11) **EP 3 335 632 B1**

(12) **EUROPEAN PATENT SPECIFICATION**

(45) Date of publication and mention of the grant of the patent:
**23.11.2022 Bulletin 2022/47**

(21) Application number: **16306672.3**

(22) Date of filing: **13.12.2016**

(51) International Patent Classification (IPC):
**A61B 5/113** $^{(2006.01)}$  **A61B 5/05** $^{(2021.01)}$
**A61B 5/0205** $^{(2006.01)}$  **A61B 5/024** $^{(2006.01)}$
**A61B 5/00** $^{(2006.01)}$

(52) Cooperative Patent Classification (CPC):
**A61B 5/1135; A61B 5/0205; A61B 5/024; A61B 5/0507; A61B 5/0816; A61B 5/7264; G16H 50/20**

(54) **METHOD OF AND APPARATUS FOR MONITORING ONE OR MORE OF A HEARTBEAT AND A RESPIRATION RATE OF A LIVE VERTEBRATE ANIMAL**

VERFAHREN UND VORRICHTUNG ZUR ÜBERWACHUNG EINES ODER MEHRERER HERZSCHLÄGE UND EINER ATMUNGSRATE EINES LEBENDEN WIRBELTIERS

PROCÉDÉ ET APPAREIL DE SURVEILLANCE D'UN OU PLUSIEURS BATTEMENTS DE C UR ET DE LA VITESSE DE RESPIRATION D'UN ANIMAL VERTÉBRÉ VIVANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**20.06.2018 Bulletin 2018/25**

(73) Proprietor: **Alcatel Lucent**
**91620 Nozay (FR)**

(72) Inventor: **Fuchs, Rolf**
**70435 Stuttgart (DE)**

(74) Representative: **DREISS Patentanwälte PartG mbB**
**Friedrichstraße 6**
**70174 Stuttgart (DE)**

(56) References cited:
**EP-A1- 2 889 804**    **DE-A1-102009 033 829**
**JP-A- 2001 017 403**    **US-A1- 2012 022 348**
**US-A1- 2016 089 052**

## Description

## Field of the invention

The disclosure relates to a method of monitoring one or more of a heartbeat of a live vertebrate animal and a respiration rate of the live vertebrate animal.

The disclosure further relates to an apparatus for monitoring one or more of a heartbeat of a live vertebrate animal and a respiration rate of the live vertebrate animal.

## Background

In medicine, monitoring is the observation of a disease, condition or one or several medical parameters over time. Monitoring can e.g. be performed by continuously measuring certain parameters by using a medical monitor (for example, by continuously measuring vital signs by a bedside monitor), and/or by repeatedly performing medical tests (such as blood glucose monitoring with a glucose meter in people with diabetes mellitus). Medical life signs monitoring of patients is necessary in hospitals, intensive care, emergency ambulance, Mobile Intensive Care Units (MICU), homes and other settings for care. Transmitting data from a monitor to a distant monitoring station is known as telemetry or biotelemetry.

Monitoring can be classified by the target of interest, including amongst others: cardiac monitoring, respiration monitoring, and so on.

Monitoring of vital parameters can include several of the aspects mentioned above, and may e.g. include blood pressure and heart rate, and preferably also pulse oximetry and respiratory rate. Multimodal monitors that simultaneously measure and display the relevant vital parameters may be integrated into bedside monitors in critical care units, and the anesthetic machines in operating rooms. These allow for continuous monitoring of a patient, with medical staff being continuously informed of the changes in general condition of a patient. Some monitors may be configured to warn of pending fatal cardiac conditions before visible signs are noticeable to clinical staff, such as atrial fibrillation or premature ventricular contraction (PVC).

A medical monitor or physiological monitor is a medical device used for monitoring. It can consist of one or more sensors, processing components, display devices, as well as communication links for displaying or recording the results elsewhere through a monitoring network.

Sensors of medical monitors may include biosensors and mechanical sensors. Conventional sensors are usually fixed at the patient's body and connected with wires to the medical monitor (for example cardiac sensors for heart rate). Other conventional sensors for respiration monitoring (sensors for flow, pressure and volume) may be built in a medical ventilator.

A major disadvantage of today's conventional medical life signs monitoring systems are the tethered sensors. The wired sensors constrain the patient and the nursing auxiliary. Based on the movement of the patient, the dependability of the monitoring with tethered sensors may be rather limited.

Conventional sensors that are positioned distant to the patient (like the pressure, volume and flow sensors of a conventional respiration monitoring device) can only insufficiently record the parameters at the patient. There is always a gap between an adjusted reference value at the machine and an actual value at the patient. Leakages of the connecting tube, the endotracheal tube or the respiratory mask between the ventilator and the patient are difficult to detect.

Incipient spontaneous breathing and cough of the patient is also difficult to detect by said conventional systems. Furthermore, a change of a patient's lung volume, e.g. caused by illness, can neither be reliably detected by the conventional systems. Moreover, state-of-the-art medical ventilators with sensors that are positioned distant to the patient can't be optimized for the individual patient. This implies a high potential risk of injuring the patient with the ventilation. Also, ventilators that are not optimized for an individual patient may extend the time needed to remove the ventilator from said patient.

As a further disadvantage of conventional medical monitoring, today there is no monitoring of a patient's movement in the bed, although this represents very important information in intensive care for example in an anesthetic recover room or when monitoring patients in coma state.

DE 10 2009 033 829 A1 discloses a method and apparatus for contactless monitoring of heart, circulation and respiration.

US 2016/089052 A1 discloses a method and device for measuring biometric data.

## Summary

The invention is set out in the appended set of claims.

Various embodiments provide an improved method of monitoring and an improved apparatus for monitoring one or more of a heartbeat of a live vertebrate animal and a respiration rate of the live vertebrate animal which do not suffer from one or more of the above-mentioned limitations.

According to the invention, the method inter alia comprises measuring a frequency spectrum of radar light reflected off the live vertebrate animal, determining amplitudes of a selected proper subset of the Fourier components of said frequency spectrum, and feeding the amplitudes of the Fourier components of the proper subset into a neural network to make a characterization of the heartbeat and/or respiration rate of the live vertebrate animal. This advantageously enables remote and contactless medical life signs monitoring (e.g. for use in intensive care) of vertebrate animals such as human beings or other mammals. Although the principle according to the embodiments is not limited to applications for human beings, in the following description, typical applications related to the medical monitoring of human beings are exemplarily provided.

In the context of the disclosure, "radar light" means electromagnetic waves that can be transmitted to a patient to be monitored, and that are at least partly reflected off the patient's body or parts thereof (e.g., the thorax), wherein at least a portion of the reflected electromagnetic waves is received and processed according to the principle of the embodiments. The reflected electromagnetic waves may also be denoted as "radar echo signal". The use of said radar light advantageously enables a remote measurement, especially without requiring any sensors or other measurement equipment to be arranged at a patient's body. Preferably, according to an embodiment, electromagnetic waves of the millimeter-wave type are used. As an example, electromagnetic waves with frequencies in the W band or the F band of the electromagnetic spectrum may be used for irradiating a patient's body. The W band of the microwave part of the electromagnetic spectrum ranges from 75 GHz to 110 GHz (Gigahertz), which corresponds with wavelengths $\lambda$ between about 4 mm (Millimeter) and about 2.7 mm. The F band ranges from 90 GHz to 140 GHz, which corresponds with wavelengths $\lambda$ between about 3.3 mm and about 2.1 mm.

Further, in the context of the disclosure, a "proper subset", which is a well-defined mathematical term, means a set which is smaller than the entire set. Thus, a proper subset of the Fourier components is less than all of the Fourier components.

The principle according to the embodiments can be used in care as well as in intensive care. Furthermore, it can be used in emergency ambulance. According to preferred embodiments, the monitored medical life signs are breathing and heartbeat activity. According to further embodiments, also the monitoring of a patient's motion is possible.

According to Applicant's analysis, the electromagnetic waves used to irradiate at least portions of the patient's body are reflected by the patient's body, particularly by the usually continuously moving chest portion and blood vessels. Additionally, said reflected electromagnetic waves may be influenced by the patient's motion features.

By analysis of the so reflected radar echo signal with algorithms according to the embodiments explained in detail further below, contactless and remote medical life signs monitoring is possible. The Doppler effect shifts and broadens the frequency of the emitted radar echo signal. While the frequency shift may be characteristic for a radial velocity of the chest and/or the blood vessels and/or the patient's motion features as seen from the radar transmitter, the spectral broadening is characteristic of internal movements of the chest, the blood vessels and the patient's motion features, such as proper motions and pulsations caused by respiration, heartbeat and body motion of the patient. The characteristic spectral broadening of the moving chest, the changing blood vessel features and the moving patient may be denoted as a "Doppler signal" and, with a proper selection of the radar frequency for the used electromagnetic waves, may produce an acoustic sound signal (e.g., a signal having frequencies within a range between about 0 Hz and about 4 kHz) as the difference between the reflected and the emitted radar echo signal. According to an embodiment, alternatively to or in addition to feeding the amplitudes of the Fourier components into a neural network, it is also possible to derive certain parameters from said Fourier components of the proper subset and to feed said parameters derived from said Fourier components into said neural network to make said characterization of the heartbeat and/or of the respiration rate (and/or of a movement) of the live vertebrate animal, which may increase a precision of the method or the characterization so obtained, respectively.

According to the invention, the measured amplitudes of the Fourier components of the selected proper subset have larger variances than the measured amplitudes of remaining ones of the Fourier components, thereby contributing to a precision of the characterization of the heartbeat and/or the respiration rate (and/or of a movement).

According to a further embodiment, different ones of the Fourier components of the selected proper subset are substantially uncorrelated over time.

According to a further embodiment, cepstral coefficients associated with said proper subset are determined and fed into said neural network. This may even further increase the precision of the characterization of e.g. the heartbeat or respiration rate (and/or of a movement) obtained via said neural network.

According to a further embodiment, the cepstral coefficients may be obtained by squaring an amplitude of respective Fourier components, by taking the logarithm of each so obtained squared Fourier component, and by performing an inverse Fourier transformation, particularly an inverse fast Fourier transformation (IFFT), on this data.

According to a particularly preferred embodiment, for each Fourier component of the selected proper subset, a corresponding cepstral coefficient is determined, and the so obtained group of cepstral coefficients (or, according to further embodiments, a selection, i.e. only a part thereof) is provided as input data to said neural network to make said characterization of the heartbeat and/or respiration rate of the live vertebrate animal.

According to yet another embodiment, a principal component analysis, PCA, is applied to said cepstral coefficients, whereby principal components associated with said cepstral coefficients are obtained, wherein said principal components are fed into said neural network.

According to a further embodiment, only some of the determined principal components are fed to the neural network.

In other words, according to some embodiments it is possible to feed said selected proper subset of the Fourier components to said neural network for characterisation. According to further embodiments, (only) cepstral coefficients obtained from Fourier components of the selected proper subset are fed to said neural network. According to other embodiments, (only) principal components as obtained by PCA based on said cepstral coefficients are fed into said neural network.

According to further preferred embodiments, combinations of one or more types of the abovementioned potential input data is fed into said neural network, e.g. amplitudes of the selected proper subset of the Fourier components and/or corresponding a cepstral coefficients and/or corresponding principal components obtained from PCA of cepstral coefficients.

According to a further embodiment, medical equipment e.g. for supporting life functions of a patient and/or for treating said patient may be controlled based on the characterization obtained according to the principle of the embodiments.

According to a method not covered by the claimed invention, a ventilator, which for example supports a breathing function of a patient, may be controlled based on the characterization obtained by said neural network.

According to the invention, an apparatus for monitoring one or more of a heartbeat of a live vertebrate animal and a respiration rate of the live vertebrate animal inter alia comprises:

a source of radar light; a light detector to measure a frequency spectrum of radar light reflected off the live vertebrate animal; a digital data processor being configured to receive said measured frequency spectrum from the light detector and to determine amplitudes of a selected proper subset of the Fourier components of said frequency spectrum; and being configured to make a characterization of the heartbeat and/or respiration rate of the live vertebrate animal based on said Fourier components of the proper subset. The digital data processor is configured such that measured amplitudes of the Fourier components of the selected proper subset have larger variances than the measured amplitudes of remaining ones of the Fourier components.

According to a further embodiment, different ones of the Fourier components of the selected proper subset are substantially uncorrelated over time.

According to an embodiment, the apparatus includes a neural network and is configured to feed the amplitudes of the Fourier components of the selected proper subset into the neural network for making said characterization of the heartbeat and/or respiration rate of the live vertebrate animal.

According to further embodiments, the apparatus may also be configured to derive one or more parameters from said Fourier components of the proper subset, and the apparatus may be configured to make a characterization of the heartbeat and/or respiration rate of the live vertebrate animal based on said Fourier components of the proper subset and/or on said one or more parameters from said Fourier components of the proper subset.

According to a further embodiment, the apparatus may be configured to feed the amplitudes of the Fourier components of the selected proper subset and/or said parameters derived from said Fourier components of the proper subset into the neural network for making said characterization of the heartbeat and/or respiration rate of the live vertebrate animal.

According to an embodiment, the apparatus is configured to determine cepstral coefficients associated with said proper subset and to feed said cepstral coefficients into said neural network.

According to an embodiment, said apparatus is configured to apply a principal component analysis, PCA, to said cepstral coefficients, whereby principal components associated with said cepstral coefficients are obtained, and to feed said principal components (or a proper subset thereof) into said neural network.

According to a further embodiment, a ventilator is provided, and the apparatus is configured to control said ventilator based on the characterization of the neural network. For this purpose, the ventilator may comprise an interface and may be configured to be controlled based on said characterization of the neural network.

**Brief description of the figures**

Further features, aspects and advantages of the illustrative embodiments are given in the following detailed description with reference to the drawings in which:

Figure 1    schematically depicts a scenario according to an embodiment,

Figure 2    schematically depicts a block diagram of an apparatus according to an embodiment,

Figure 3A    depicts a simplified flow-chart of a method according to an embodiment,

Figure 3B    depicts a simplified flow-chart of a method according to a further embodiment,

Figure 4    schematically depicts a scenario according to a further embodiment,

Figure 5    schematically depicts a spatial arrangement of components according to an embodiment,

Figure 6A    schematically depicts a block diagram of a signal processing chain according to an embodiment,

Figure 6B    schematically depicts a block diagram of details of the signal processing chain according to Figure 6A,

Figure 7    schematically depicts a topology of a neural network according to an embodiment,

Figure 8    schematically depicts a table illustrating exemplary output data of a neural network according to an embodiment,

Figure 9A    schematically depicts a block diagram of a signal processing chain according to a further embodiment,

Figure 9B    schematically depicts a block diagram of a signal processing chain according to a further embodiment, and

Figure 10    schematically depicts a respiration pressure curve over time according to an embodiment.

## Description of the embodiments

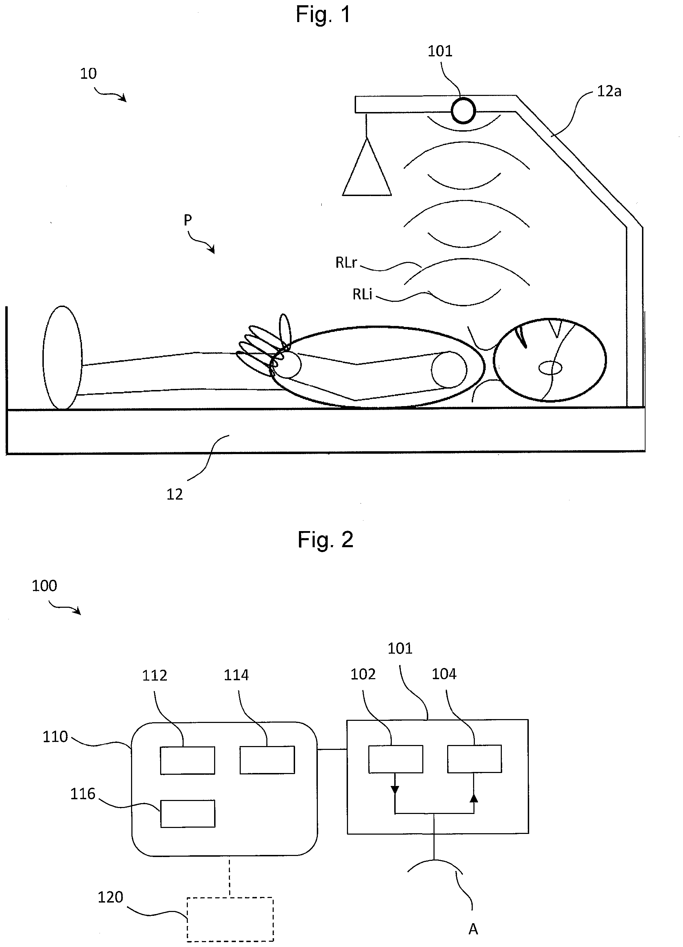

Figure 1 schematically depicts a scenario 10 according to an embodiment. Depicted is a hospital bed 12 with a patient P that is subject to contactless monitoring of a heartbeat and/or a respiration rate. For this purpose, a source of radar light is provided above the patient P, which emits radar light upon an upper part of the body, cervical and head of the patient P. The radar light irradiated onto the patient's body is denoted with reference sign RLi, and radar light reflected off the patient's body is denoted with reference sign RLr.

At least a portion of the reflected radar light RLr is received by a detector to measure a frequency spectrum of said reflected radar light RLr. According to the present embodiment, both the source of radar light and the detector are arranged in a common device 101 which is attached to a support 12a which forms a part of the hospital bed 12.

Alternatively, the device 101 may be mounted to a ceiling (not shown) or any other support structure around the bed 12 offering a direct line-of-sight transmission of said irradiated radar light RLi and said reflected radar light RLr. According to further embodiments, it is possible that the source of radar light and the detector, which receives the reflected radar light RLr, are not co-located, but rather placed in different positions around the patient P, as long as respective direct line-of-sight transmission paths are provided between the radar source and the patient P and the patient P and the detector.

With the configuration presented by Fig. 1, a contactless monitoring of a heartbeat and/or a respiration rate and/or of body movements of the patient P is enabled. Fig. 2 schematically depicts a block diagram of a corresponding apparatus 100 for monitoring one or more life signs of a live vertebrate animal according to an embodiment.

The apparatus 100 comprises the device 101 already explained above with reference to figure 1. As can be seen from figure 2, the device 101 comprises a source 102 of radar light and a light detector 104 to measure a frequency spectrum of radar light reflected off the live vertebrate animal, presently the patient P (Fig. 1). As an example, the device 101 may be configured to emit a sequence of comparatively short radar signals at a pulse frequency of about 4 kHz, wherein a carrier frequency of the radar signals is within a frequency range corresponding to the W band or the F band. As an example, the carrier frequency may be 100 GHz.

In the present example, for this purpose, the source 102 is configured to generate said pulsed periodic signal with the carrier frequency of 100 GHz, and the device 101 comprises circuitry for transmitting said pulsed periodic signal to the antenna A, which is provided for irradiating the object under measurement, presently the patient P (Fig. 1), with the radar light. After a certain retention period, which e.g. depends on the distance between the device 101 or its antenna A and the reflecting structures of the body of the patient P, the antenna A receives a reflected Doppler-broadened radar echo signal, which is forwarded by further circuitry of the device 101 to the detector 104. According to an embodiment, the circuitry for providing the RF output signal of source 102 to the antenna A and for providing a received radar echo signal from the antenna A to the detector 104 comprises at least one directional coupler (not shown).

The apparatus 100 further comprises a digital data processor 112 which is configured to receive said measured frequency spectrum from the light detector 104 and to determine amplitudes of a selected proper subset of the Fourier components of said frequency spectrum. Based on this selected proper subset of the Fourier components of said frequency spectrum, the apparatus 100 may make a characterization of a heartbeat and/or a respiration rate of a live vertebrate animal such as the patient P (Fig. 1)

According to an embodiment, the processor 112 comprises a digital signal processor (DSP) or general purpose microprocessor or correspondingly configured (and/or reconfigurable) field programmable gate array (FPGA) or an ASIC (application specific integrated circuit) or the like.

According to a further embodiment, a digital data memory 114 may be provided which may be accessed by the digital data processor 112 for at least temporarily storing data, such as data to be processed, e.g. data related to the measured frequency spectrum from the light detector 104 and/or the determined amplitudes of a selected proper subset of the Fourier components of said frequency spectrum.

According to a further embodiment, the memory 114 comprises a RAM (random access memory) and/or ROM (read only memory) and/or other forms of volatile and/or non-volatile memory devices. In one or more memory devices of the digital data memory 114, a computer program for executing the steps according to one or more of the present embodiments may be provided.

According to further embodiments, the digital data memory 114 is at least partly co-located with the digital data processor 112. The digital data memory 114 may e.g. be at least partly be integrated to said processor 112 or arranged together with said processor 112 on a common substrate such as a common printed circuit board. According to further embodiments, at least parts of said digital data memory may also be arranged external to the apparatus 100 (Fig. 2) or remote from said apparatus 100.

According to an embodiment, at least a part of the memory 114 may be implemented in the form of cloud-based storage, said cloud based storage being provided within a data network such as the Internet, which may e.g. be accessed by the apparatus 100 via an Internet connection.

Advantageously, the processor 112 and the (optional) memory 114 form a combination 110 which facilitates characterizing the heartbeat and/or a respiration rate of a live vertebrate animal.

According to a further embodiment, said apparatus 100, or, according to further embodiments, said combination 110 comprises a neural network 116 and is configured to feed the amplitudes of the Fourier components of the selected proper subset into the neural network 116. The neural network 116 is configured to make a characterization of the heartbeat and/or respiration rate and/or a movement of the body of the patient P based on the fed amplitudes of the Fourier components.

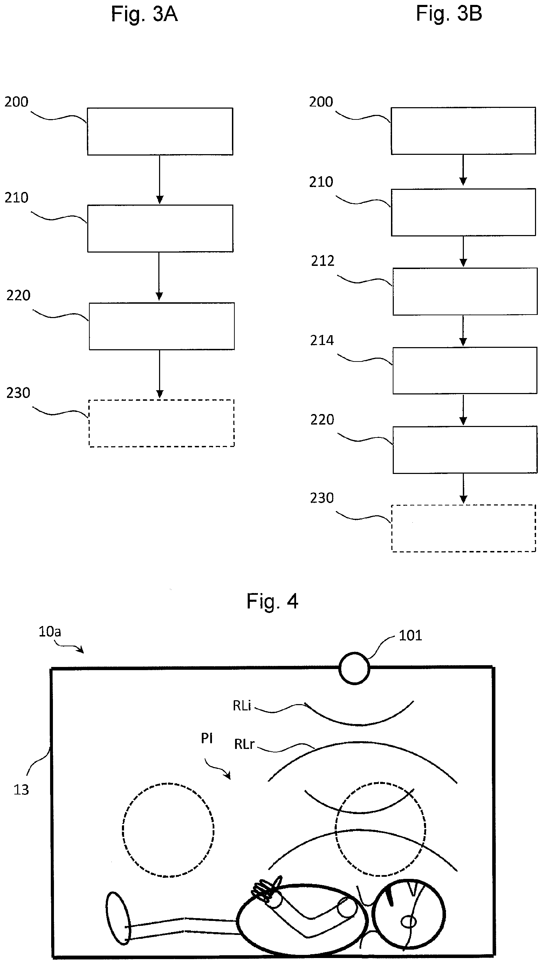

Figure 3A depicts a simplified flowchart of a method of monitoring according to an embodiment. In a first step 200, a frequency spectrum of radar light RLr (Fig. 1) reflected off a live vertebrate animal is measured. This is for example performed by the detector 104 as explained above with reference to figure 2.

In a subsequent step 210 (Fig. 3A), amplitudes of a selected proper subset of the Fourier components of said frequency spectrum are determined. Said step 210 is e.g. executed by the combination 110 of the data processor 112 and the data memory 114.

According to the invention, the measured amplitudes of the Fourier components of the selected proper subset have larger variances than the measured amplitudes of remaining ones of the Fourier components. Further preferably, different ones of the Fourier components of the selected proper subset are substantially uncorrelated over time.

After step 210 (Fig. 3A), a further step 220 is performed which is directed to feeding the amplitudes of the Fourier components of the proper subset into said neural network 116 (Fig. 2) to make a characterization of the heartbeat and/or respiration rate and/or a body movement of the live vertebrate animal, presently the patient P (Fig. 1).

The above explained steps 200, 210, 220 provide an efficient and precise contactless medical life signs monitoring using millimeter-wave radar.

Optionally, as indicated by the further step 230 (Fig. 3A), a ventilator 120 (Fig. 2) may be provided which may be controlled based on the characterization provided for by said neural network 116. Thus, a closed-loop control of the ventilator 120 is enabled which individually supports a breathing function of the patient P depending on his life signs monitored according to the embodiments by means of steps 200, 210, 220.

Figure 3B depicts a simplified flowchart of a method of monitoring according to a further embodiment. In a first step 200, a frequency spectrum of radar light RLr (Fig. 1) reflected off a live vertebrate animal is measured. This is for example performed by the detector 104 as explained above with reference to figure 2.

In a subsequent step 210 (Fig. 3B), amplitudes of a selected proper subset of the Fourier components of said frequency spectrum are determined. Said step 210 is e.g. executed by the combination 110 of the data processor 112 and the data memory 114.

In subsequent step 212, cepstral coefficients associated with said proper subset are determined. According to an embodiment, the cepstral coefficients may be obtained by squaring an amplitude of respective Fourier components, by taking the logarithm of each so obtained squared Fourier component, and by performing an inverse Fourier transformation, particularly an inverse fast Fourier transformation (IFFT), on this data. Presently, in step 212, for each Fourier component of said selected subset, a corresponding cepstral coefficient is determined.

After this, in step 214, a principal component analysis, PCA, is applied to said cepstral coefficients as obtained within preceding step 212, whereby principal components associated with said cepstral coefficients are obtained.

Further, in step 220, said principal components are fed into said neural network 116 (Fig. 2).

According to Applicant's analysis, performing the steps 212, 214 and feeding the so obtained principal components of the cepstral coefficients into the neural network 116 may yield an even more precise characterization of the heartbeat and/or respiration rate and/or a body movement of the live vertebrate animal - as compared to the process of Fig. 3A.

According to further embodiments, combinations of one or more types of the abovementioned potential input data is fed into said neural network 116 in step 220, e.g. amplitudes of the selected proper subset of the Fourier components and/or corresponding a cepstral coefficients and/or corresponding principal components obtained from PCA of cepstral coefficients.

As with the embodiment of Fig. 3A, in the process of Fig. 3B, after step 220, an optional step 230 of controlling a ventilator 120 (Fig. 2) may follow according to some embodiments.

According to a further embodiment, only some of the determined principal components are fed to the neural network, e.g. only a selected proper subset of said principal components.

According to preferred embodiments, the steps 210, 212, 214, 220, 230 may be carried out by the combination 110 with the processor 112 and the memory 114, for example by means of a computer program stored to said memory 114, wherein instructions of said computer program are processed by the processor 112.

Figure 4 schematically depicts a scenario 10a according to a further embodiment. Depicted is a premature infant PI in an incubator 13. Similar to the scenario 10 of Fig. 1, in this situation, too, the method and apparatus according to the embodiments may be used for monitoring the heartbeat and/or a respiration rate and/or a body movement of the premature infant PI. For this purpose, the device 101, which may also be termed "radar sensor" as it is capable of transmitting the radar light RLi and of receiving reflected radar light RLr, is arranged on a top cover of the incubator 13, above an upper part of the body, cervical and head of the infant. Advantageously, in this setting, too, a precise remote and contactless measurement of the heartbeat and/or a respiration rate and/or a body movement of the premature infant PI is enabled by the principle according to the embodiments.

Optionally, in this situation 10a, too, it is possible to control medical equipment (not shown) e.g. for supporting life functions of the infant and/or for treating said infant based on the characterization obtained by means of said neural network 116 (Fig. 2).

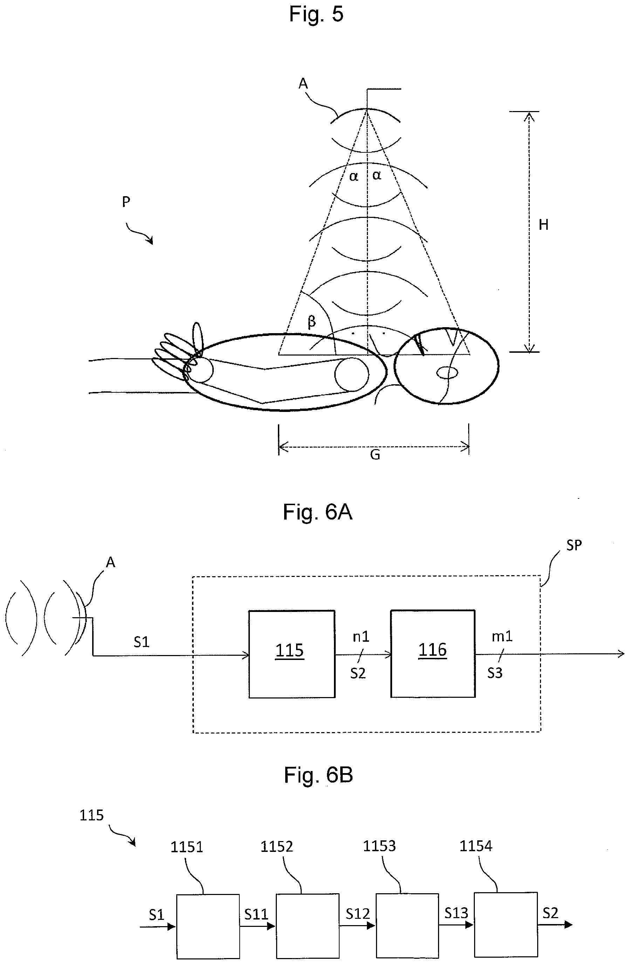

Figure 5 schematically depicts a spatial arrangement of components according to an embodiment. Preferably, the arrangement of the radar antenna A and its antenna characteristic are chosen such that an upper part of the body, the cervical and head are within a horizontal detection range G of said antenna A, wherein the antenna A is positioned at a given vertical distance H from the patient's body.

The following equation shows the trigonometry functions to calculate the spatial arrangement of the radar antenna A to the patient P according to an embodiment: $\tan \alpha = G/(2*H)$, wherein "*" is the multiplication operator. With the help of the trigonometry functions indicated in the following equation it is possible to calculate the angle $(2*\alpha)$ of radiation for different applications: $2*\alpha = \arctan (G/H)$.

As an example, the angle of radiation for usage of the apparatus according to the embodiments with a hospital bed (distance H between the radar sensor 101 or antenna A and the patient of 1.0m) is approximately $2*\alpha=40°$, and for the usage with an incubator 13 (cf. Fig. 4, distance H between the radar sensor 101 and the premature infant PI of 0.6m) approximately $2*\alpha=34°$.

Figure 6A schematically depicts a block diagram of a signal processing chain SP according to an embodiment. An input signal S1 is the Doppler broadened radar echo signal from the radar antenna A, an output signal S3 is the output of the monitored medical life signs.

Radar light reflected off a patient P (Fig. 1) is received by the antenna A (Fig. 6A) and fed to a preprocessing stage 115 as an input signal S1. Details of the preprocessing stage 115 according to an embodiment are provided by Fig. 6B. The preprocessing stage 115 may e.g. be implemented by the combination 110, especially a computer program executed by the processor 112.

In the preprocessing stage 115, according to an embodiment, a feature extraction of the Doppler broadened radar echo signal S1 is carried out. For this purpose, the Doppler broadened radar echo signal S1 is demodulated by demodulator 1151 (Fig. 6B), whereby a demodulated signal S11 is obtained at an output of the demodulator 1151.

According to an embodiment, the demodulation may e.g. be effected by downconverting the signal S1 to a baseband frequency range in a per se know manner. As an example, the signal S1 may be multiplied with a reference signal obtained from the source 102 (Fig. 2), which effects a transformation of said signal S1 into a baseband frequency range. As a further example, if electromagnetic waves with a frequency of 100 GHz are used as radar light RLi (Fig. 1) for irradiating a patient's body, and if said radar light RLi is pulsed with a pulse frequency of e.g. 4 kHz, for said step of demodulating, the signal S1 may be multiplied with a periodic (e.g., sinusoidal) reference signal of 100 GHz, resulting in a downconverted signal the substantial amount of signal energy of which is located within a frequency range of about 0 Hz to about 4 kHz.

In other words, the demodulation "removes" the carrier frequency component of the emitted radar signal from the input signal S1. The result is a time spectrum with a pulse period that corresponds to the pulse frequency rate of the emitted radar signals (4 kHz).

According to an embodiment, the time spectrum within each pulse period is divided into a number of time windows which are representative of a predetermined average distance as a function of the running time of the radar light. The number of time windows corresponds to a distance resolution and depends on the pulse duration of the emitted radar signals.

In other words, according to a preferred embodiment, the demodulated signal S11 may be windowed in the time domain by block 1152, whereby a windowed signal S12 is obtained at an output of a block 1152. Said step of windowing may e.g. be performed by multiplying the downconverted signal with a predetermined windowing function.

According to a preferred embodiment, the demodulated signal S11 may also be converted into a digital signal by means of an analog to digital (A/D) converter prior to said windowing. In this case, said A/D converter (not shown) may be integrated in block 1151, and the output signal S11 of block 1151 comprises a first number of sample values. In other words, in this case, the output signal S11 is a time-discrete signal also having discrete amplitude values. The further explanation of the preprocessing stage 115 refers to this configuration. Alternatively, the A/D conversion may be performed after said windowing 1152.

According to a preferred embodiment, the demodulated signal S11 is sampled with a frequency corresponding to the pulse frequency of the emitted radar signals, for example with 4 kHz.

As an example, when windowing said a digital downconverted signal such as signal S11, a second number of digital sample values is obtained, which second number is smaller than said first number and which depends on a sample rate and the length of the window used for said step of windowing. As a further example, said number is 256. These sample values may also be interpreted as a signal vector S12.

After the windowing process, the windowed signal S12 is mapped into the frequency domain, for example with the help of an FFT (Fast Fourier Transformation), cf. function block 1153 of Fig. 6B. As a result, Fourier components (or FFT coefficients, respectively), for example 256 many Fourier components, are obtained at an output of FFT block 1153, which may also be interpreted as an FFT vector S13.

In a subsequent step, cf. block 1154, a proper subset of said 256 many Fourier components is selected. Said selected proper subset is provided as an output signal S2 at the output of block 1154.

According to a preferred embodiment, the measured amplitudes of the Fourier components of the selected proper subset S2 have larger variances than the measured amplitudes of remaining ones of the Fourier components.

According to a further embodiment, different ones of the Fourier components of the selected proper subset S2 are substantially uncorrelated over time.

According to a further embodiment, said selected proper subset S2 of Fourier components comprises those Fourier components of the FFT vector S13, which are associated with positive frequencies and a zero position, i.e. a DC component of the discrete signal spectrum represented by the FFT vector S13. As an example, based on an FFT vector S13 comprising 256 many Fourier components, according to the present embodiment, the selection of the proper subset effects that said selected proper subset S2 comprises e.g. n1=129 Fourier components.

According to a further embodiment, the selection within block 1154 of Fig. 6B may also be performed such that said selected proper subset S2 of the Fourier components comprises less than 128 Fourier components. As an example, it may be considered that 64 Fourier components are selected, e.g. by disregarding 64 Fourier components which are associated with higher signal frequencies.

According to further embodiments, other approaches are also possible for obtaining said selected proper subset of the Fourier components.

According to Applicant's analysis, performing the selection step of block 1154 which leads to said selected proper subset S2 (Fig. 6A) of n1 many Fourier components according to the embodiments advantageously improves a further processing by the neural network 116 according to the principle of the embodiments.

According to an embodiment, the signal S2 representing n1 many Fourier components, or their amplitudes, respectively, of the selected proper subset as obtained by block 1154 of the preprocessing stage 115, is output to the neural network 116, cf. Fig. 6A. On this basis, the neural network 116 makes a characterization of a heartbeat and/or respiration rate and/or body movement of the patient P (Fig. 1) from whom the radar echo signal S1 input to the preprocessing stage 115 has been obtained.

According to further embodiments, alternatively to or in addition to providing said selected proper subset S2 of the Fourier components to the neural network 116, other parameters may be derived from said Fourier components of the selected proper subset, which other parameters may be fed to the neural network 116 for performing said characterization of a heartbeat and/or respiration rate and/or body movement of the patient P.

According to an embodiment, cepstral coefficients associated with said selected proper subset S2 are determined and fed into said neural network 116. As an example, the determination of the cepstral coefficients may e.g. be performed by the function block 1154 of figure 6B.

According to a further embodiment, the cepstral coefficients may be obtained by squaring an amplitude of each Fourier component, by taking the logarithm of each so obtained squared Fourier component, and by performing an inverse Fourier transformation, particularly an inverse FFT (IFFT), on this data. This resulting data is called cepstrum, and the coefficients are called cepstral coefficients.

According to an embodiment, the representation of information comprised in the received Doppler signal S1 as a cepstrum is particularly advantageous because this enables a particularly reliable classification using the neural network 116.

According to yet another embodiment, a principal component analysis, PCA, is applied to said cepstral coefficients, whereby principal components associated with said cepstral coefficients are obtained, wherein said principal components are fed into said neural network 116. According to a preferred embodiment, only n many first principal components as obtained from said PCA may be fed into said neural network 116.

With the mathematical procedure of the PCA the cepstral coefficients (which may represent possibly correlated variables) are transformed into a (smaller) set of values of linearly uncorrelated variables called principal components. This transformation is defined in such a way that the first principal component has the largest possible variance (that is, accounts for as much of the variability in the data as possible), and each succeeding component in turn has the highest variance possible under the constraint that it be orthogonal to (i.e., uncorrelated with) the preceding components. The first principal components are the most important ones, which explain the highest part of the variance, thus comprising the highest content of information of the signal so analyzed. According to an embodiment, the objective of the PCA is to reduce the comparatively complex data set, e.g. of the cepstrum, to a lower dimension to reveal hidden, simplified structures that often underlie it. After the PCA, the neural network 116 can make the required characterizations based on comparatively few variables which - however - represent the main part of the variance of the input signal S1.

According to further aspects, depending on the field of application, the PCA is also named the discrete Karhunen-Loeve transform (KLT), the Hotelling transform or proper orthogonal decomposition (POD).

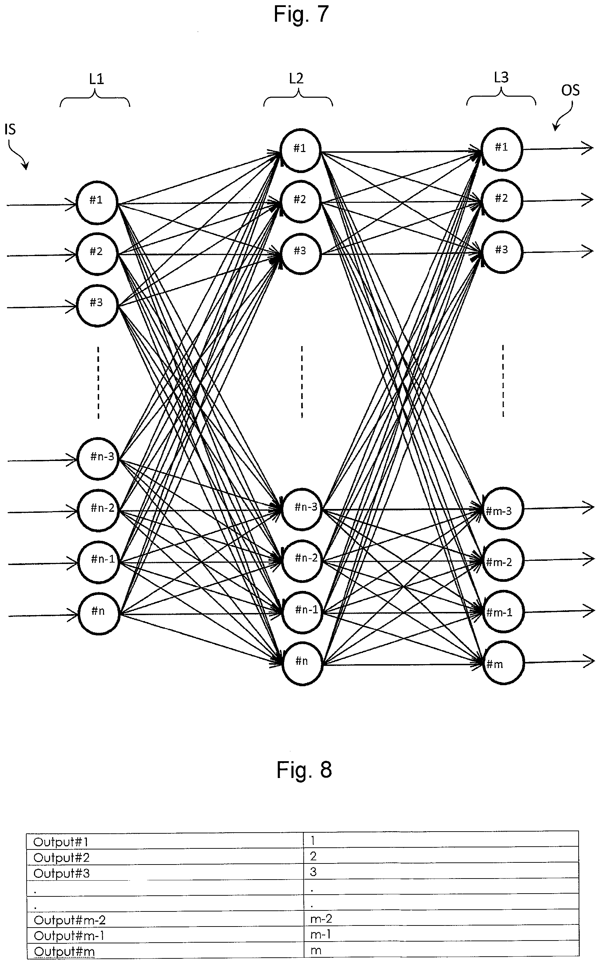

According to a further embodiment, the first n1 principal components as obtained from the PCA are presented to the neural network 116 (Fig. 6A) as input data S2 to n many input nodes, cf. the explanations related to Fig. 7 below. As mentioned above, preferably, only the first n1 many principal components are selected as input to the neural network 116, as the further (upper) ones are comparatively insignificant based on the small part of their variance. As an example, with a cepstrum of 256 elements (i.e., cepstral coefficients) the corresponding principal components n1 to 128 of the digital complex baseband signal receive no further consideration because n many coefficients of the digital complex baseband signal are sufficient for a reliable characterization of the input signal S1 by means of the neural network 116. At an output of said neural network 116, m1 many output values are obtained representing the output signal S3.

A neural network comprises nodes and synaptic connections between the nodes. Weights which are determined by training with training data are allocated to the synaptic connections. The nodes indicate an activity as the result of input data. The neural network is subdivided into different hierarchical layers, where every node of a lower layer is connected to every node of a higher layer through synaptic connections. A neural network with such a topology is called a multi-layer perceptron. The input data for the nodes is the activation of the nodes in the next lower layer, multiplied by the weight of the respective synaptic connection. In the following equation one can see the activation $y_n$ as result of a node $N_n$ of a

$$y_n = f(\sum_i w_{in} x_i)$$

neural network:                                                    ,

wherein f() is a predetermined function, $x_i$ is the activation of the $i_{th}$ node $N_i$, and $w_{in}$ is the weight of the synaptic connection between the $i_{th}$ node $N_i$ and the nth node $N_n$.

According to an embodiment, the input data of the lowest layer for said neural network 116 (Fig. 6A), called the input layer, are features of the Doppler broadened radar echo signals, e.g. in the form of said selected proper subset S2 of Fourier components and/or parameters derived therefrom such as the cepstral coefficients or the principal components obtained by PCA (or proper subsets thereof). According to an embodiment, the output signal S3 of the neural network 116 represents the heartbeat, e.g. a heart rate, and/or the breathing rate, and/or the moving activity rate.

According to an embodiment, the classification result of the neural network is determined by the activation of the nodes in the uppermost layer called the output layer. According to a further embodiment, the greatest probability is that a dedicated breathing rate, heart rate or the moving activity rate belongs to the class whose output node exhibits the highest activation.

Figure 7 schematically depicts a topology of a neural network according to an embodiment. For example, the neural network 116 of Fig. 6A may comprise the structure as depicted by Fig. 7.

The neural network 116 comprises three layers L1, L2, L3, wherein a first layer L1 is an input layer, a second layer L2 is an intermediate layer, also denoted as hidden layer, and wherein a third layer L3 is an output layer.

According to a preferred embodiment, all nodes of one layer have synaptic connections with all nodes of the next layer. The input layer L1 has n many input layer nodes (#1 to #n), and each of them has an input indicated by a respective arrow. The hidden layer L2 may also have n many nodes (#1 to #n), as depicted by Fig. 7, but may according to further embodiments also have a different number of nodes, e.g. more or less than n many nodes.

As already mentioned above, according to preferred embodiments, the output data of the preprocessing stage 115 (Fig. 6A) is fed to the neural network 116. As a consequence, features extracted from the Doppler broadened radar Echo signals by means of said preprocessing stage 115 are used as input signals IS to the input layer L1 of the neural network 116.

According to the present embodiment, n many input nodes are provided, wherein each of said input nodes of the input layer L1 comprises synaptic connections with all nodes of the hidden layer L2. According to the present embodiment, the neural network 116 has only one hidden layer L2. However, according to further embodiments, it is also possible to provide more than one hidden layer. When providing more than one hidden layer, classification results of the neural network may be more precise as compared to one single hidden layer. However, the computing time of the neural network is strongly growing with each additional hidden layer. Thus, a particularly preferred embodiment comprises one single hidden layer for the neural network 116.

According to an embodiment, the number n of input signals to the neural network depends on the information value of the input parameters. With an additional preprocessing transformation of the set of observations of correlated variables into a set of values of linearly uncorrelated variables, the classification result can be significantly enhanced. In this context, said determination of cepstral coefficients and/or principal components of said cepstral coefficients and feeding this data (or a proper subset of said cepstral coefficients and/or said principal components) into the neural network 116 is particularly beneficial.

According to a further embodiment, the number n of input signals IS to the neural network 116 may be reduced, while keeping constant or enhancing the quality of the classification result of the neural network 116. According to a preferred embodiment, for using the neural network 116 for a classification of a heart rate and/or a breathing rate and/or a moving activity rate of the patient P, the number n of input signals may be chosen to be in the range of about 30.

According to further embodiments, more than 30 input signals IS may also be used for the classification.

However, again, the processing complexity for the neural network 116 increases. At an output of the neural network, output signals OS (Fig. 7) are provided, presently in the form of m many outputs, corresponding to the number m of nodes of the output layer L3. In Fig. 6A, the output data of the neural network 116 is indicated with reference sign S3.

Figure 8 depicts a table that shows an exemplary mapping of the output layer classifications of the neural network according to Fig. 7 to the breathing rate or heart rate per minute or moving activity rate (per minute) of the patient.

According to an embodiment, a classification or characterization of data performed by the neural network comprises storing classification results and using such classification results stored in the past to make conclusions about future input data and their classification or characterization.

According to a further embodiment, the neural network 116 (Fig. 2) may learn with training data from sets of different outputs. In this case the training data are presented to the neural network often enough, until the neural network has learned to allocate all training data correctly by varying the weights of the synaptic connections. According to a further embodiment, the training data may also be preprocessed to cepstral coefficients and/or principal components as described above. Preferably, the training of the neural network takes place in accordance with an algorithm, such as for example the error back propagation algorithm.

According to a further embodiment, during a training process, all the output nodes are trained by themselves. This denotes that the neural network was trained with training data from all Doppler broadened radar echo signals, until the neural network was able to allocate all the training data to the right class.

Advantageously, according to a further embodiment, the weights of the synaptic connections, which were determined once through training, can be read out after the training to reproduce the topology of the neural network any number of times. This may be beneficial when providing a plurality of apparatuses 100 (Fig. 2) according to the embodiments. For example, only a single apparatus - or the neural network 116 of said apparatus - is required to be trained. After this, the so obtained data, i.e. the weights of the synaptic connections learned during training, may be transferred to the neural network of a further apparatus, so that no (further) training is required. Therefore, not every individual apparatus 100 requires training.

According to a further embodiment, another training possibility for the neural network is to use the algorithm for cascade correlation described in https://de.wikipedia.org/wiki/Cascade_Correlation.

According to this method, the neural network may be produced during the training. This denotes that the training establishes both the weights of the synaptic connections as well as the topology (i.e., number of nodes within the different layers, their synaptic connections, and the like) of the neural network.

According to a further embodiment, since it is not possible to train a neural network with training data for all (theoretically) possible conditions and all possible combinations of different influence factors with different conditions, it is proposed to use a neural network which is adapted to the conditions prevailing at the time the Doppler broadened radar echo signals and hence e.g. the calculated principal components are determined. In other words, according to some embodiments the neural network may be trained with conditions/influence factors that are expected to be relevant later during measurements, e.g. when the apparatus is used in the field.

According to a further embodiment, the neural network(s) of the apparatus may be trained with male and female probands of all age groups, e.g. from a baby over a child, an adult to an old person. In this case, the apparatus can be used for detection of one or more of e.g. a heartbeat of male and female persons of all ages.

According to further embodiments, if the apparatus is intended to be used only for a specific, limited field of application, such as for an incubator for premature infants, it may not be necessary to train the neural network with adults or old persons.

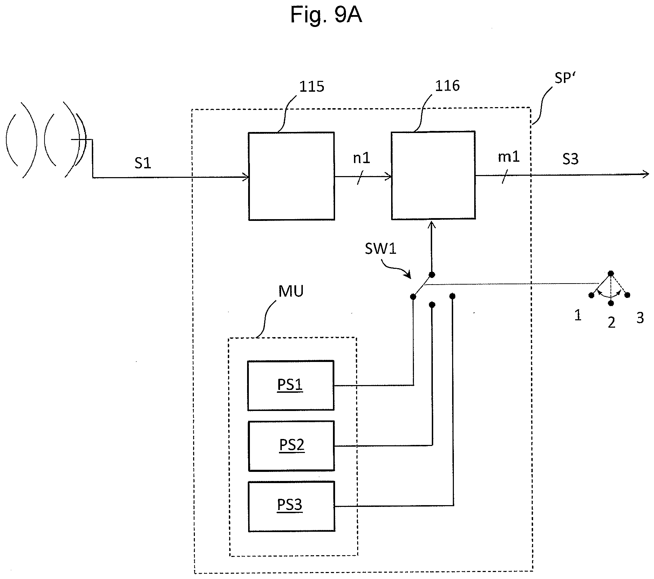

According to a further embodiment, the signal processing chain of the apparatus according to the embodiments may be provided with an additional memory unit MU. Figure 9A schematically depicts a simplified block diagram of such an embodiment SP'.

According to the embodiment, the memory unit MU serves to store different parameter sets, presently three different parameter sets PS1, PS2, PS3, which may selectively be applied to the neural network 116, as symbolically indicated by the switch SW1 and its associated three switching positions "1", "2", "3".

According to an embodiment, said memory unit MU may form part of the digital data memory 114 explained above with reference to Fig. 2. According to furthers embodiments, at least partly, said memory unit MU may be implemented by any form of cloud storage, which efficiently enables to provide said different parameter sets to a plurality of apparatuses according to the embodiments.

The depicted configuration advantageously enables a combined detection of several life signs such as breathing activity, heartbeat activity and monitoring of the patient's motion.

As an example, each of said three parameter sets PS1, PS2, PS3 may have been determined from training under different sets of conditions of different life signs and external influence factors or a combination out of it. As a further example, the first parameter set PS1 may represent a set of parameters trained (and optimized) for characterization of a heartbeat of a patient, while the second parameter set PS2 may represent a set of parameters trained (and optimized) for characterization of a respiration rate of a patient, and the third parameter set PS3 may represent a set of parameters trained (and optimized) for characterization of a body movement of a patient.

Hence, if the heartbeat is to be characterized, the first parameter set PS1 is applied to neural network 116 by controlling the switch SW1 to its position "1", and once this parameter set is loaded, the neural network 116 is ready for characterizing heartbeat in an optimized fashion depending on input data S1 fed from the preprocessing stage 115. If a respiration rate or body movement is to be characterized, the other parameter sets may correspondingly be applied. Altogether, this embodiment enables a precise characterization of the different vital signals of a patient, for example in a time-multiplexed manner, e.g. applying a round-robin scheme.

According to an embodiment, the various parameter sets PS1, PS2, PS3 may be generated with training data under different external conditions of a definite life sign. The sets of parameters e.g. contain the weights of the individual synaptic connections for the neural network.

According to a further embodiment, the switch SW1 can be controlled automatically, e.g. by the apparatus 100 (Fig. 2), or manually, e.g. by a user of the apparatus 100, such as a nursing auxiliary. As an example, in case of certain circumstances, the manual control of switch SW1 can be made with separate individual setting options in a service approach of the equipment.

According to a further embodiment, a computer program may be provided for execution by the processor 112 (Fig. 2) to implement the neural network 116. In this case, the desired parameter set may be loaded for use with the neural network. For example, a RAM memory accessible by the processor 112 may be used for at least temporarily storing the neural network and an active parameter set.

According to a further embodiment, it is also possible to use a Field Programmable Gate Array (FPGA), preferably with partial reconfiguration functionality, to implement the neural network 116. The partial reconfiguration functionality facilitates an exchange of parameter sets for the neural network and provides a fast and on the fly changing of the "software", which is particularly useful if the parameter sets are frequently changed.

According to further embodiments, it is also possible to provide different parameter sets for different types of patients to be monitored. As an example, a first parameter set may be provided for heartbeat characterization of adults, while one or more further parameter sets may be provided for heartbeat characterization of children or premature infants.

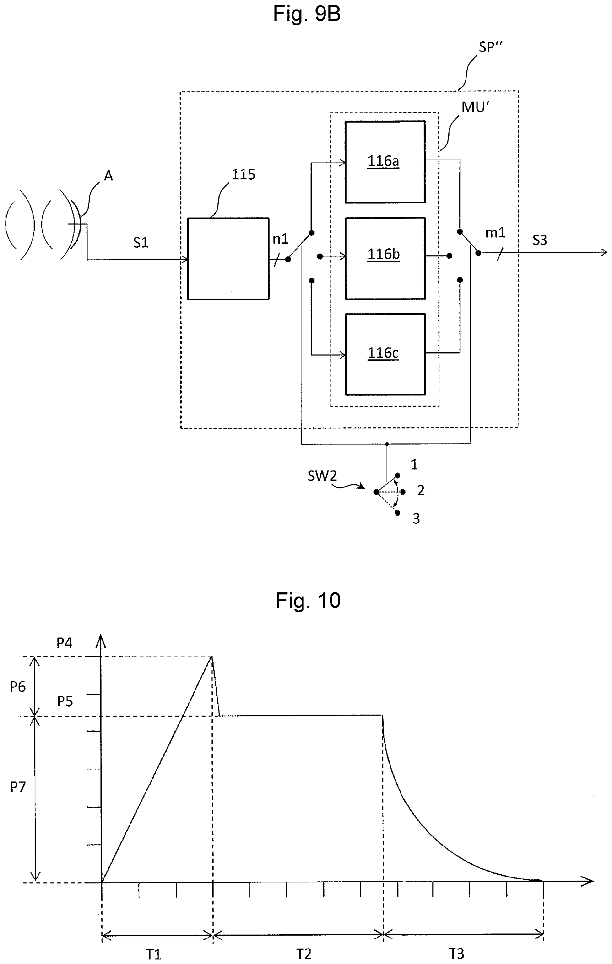

Figure 9B schematically depicts a simplified block diagram of a signal processing chain SP" according to a further embodiment. In contrast to changing parameter sets PS1, PS2, PS3 as explained above with reference to Fig. 9A, the configuration according to Fig. 9B provides three entire topologies of different neural networks, cf. the reference signs 116a, 116b, 116c.

A memory unit MU' is provided, which stores the entire topologies of the three different neural networks 116. This may be particularly beneficial if instead of a multi-layer perceptron-type network, a neural network of the cascade-correlated networks type, whose topology is only determined by the training, is used for the classification. In this case, in the memory unit MU' said three entire topologies of different neural networks 116a, 116b, 116c are stored.

The switch SW2 enables to select a specific one of said three different neural networks 116a, 116b, 116c, depending on its switching state. For example, the first neural network 116a (switching state "1") may be selected for characterizing heartbeat activity, the second neural network 116b (switching state "2") may be selected for characterizing breathing activity (e.g., respiration rate), and the third neural network 116c (switching state "3") may be selected for characterizing motion of the patient P (Fig. 1). Each of said three neural networks 116a, 116b, 116c may be especially trained for characterizing the respective life sign and external influence factors or a combination thereof. According to an embodiment, such influence factors can be dialects. As an example, the selection considers the language and the external conditions at the prevailing time.

According to a further embodiment, the switch SW2 can be controlled automatically, e.g. by the apparatus 100 (Fig. 2), or manually, e.g. by a user of the apparatus 100, such as a nursing auxiliary. As an example, in case of certain circumstances, the manual control of switch SW2 can be made with separate individual setting options in a service approach of the equipment.

According to a preferred embodiment, a complete software code and/or variables and/or other parameters of all three neural networks 116a, 116b, 116c with the corresponding parameter sets may be provided in a main memory, e.g. RAM, which is accessible by the processor 112 (Fig. 2).

By actuating the switch SW2, the required neural network of the selected definite life sign can be used. An embodiment with the storage of the entire topologies of different neural networks 116a, 116b, 116c is particularly advantageous for fast changing between the definite life signs.

Furthermore, also with the configuration of Fig. 9B, it is possible to use an FPGA, preferably with partial reconfiguration. If a frequent change of the neural network is necessary, this approach provides a fast and on the fly changing of the software implementing the neural network.

According to further embodiments, it is also possible to provide neural networks (with the corresponding parameter set) for different types of patients and/or different external conditions of the definite life sign to be monitored. As an example, a first neural network may be provided for heartbeat characterization of adults, while one or more further neural networks may be provided for heartbeat characterization of children or premature infants.

According to an embodiment, the apparatus 100 (Fig. 2) may be configured with cross-linked microprocessors operating in parallel. A possible alternative is a configuration with only one microprocessor 112 which operates the individual nodes of the network sequentially. In this case, a buffer memory may be provided for storing intermediate results during evaluation of the neural network.

The embodiments enable a remote and contactless medical life signs monitoring. The safety and quality of medical care in hospitals, intensive care, emergency ambulance, Mobile Intensive Care Units (MICU), homes and other settings for care can be significantly enhanced by using the embodiments.

The principle according to the embodiments works remotely and contactless with an extremely fine range resolution (in the millimeter range), which enables a particularly precise characterization of measured reflected radar light. The superior range resolution is also given when transmitting the used electromagnetic waves through clothes.

As a further example, when using the principle according to the embodiments, it is possible to optimize an operation and/or settings of a medical ventilator (cf. reference sign 120 of Fig. 2) used for supporting a breathing function in all phases of ventilation.

Further advantageously, using the principle according to the embodiments, a movement of the patient can be monitored. This is very important information in intensive care for example in an anesthetic recover room or to monitoring of patients in coma state.

Further, for the life signs monitoring according to the embodiments, no tethered sensors are necessary. On this account no wired sensors constrain the patient and the nursing auxiliary. Moreover, there are no sensors necessary that are fixed at the patient's body.

Still further, the method according to the embodiments enables the measurement of the actual values remote and contactless directly at the patient. The record of measurements direct at the patient is exact and reliable. For example, by applying the principle according to the embodiments, the respiration monitoring and artificial respiration can be made much safer. There are no gaps between adjusted reference values at a medical ventilator and an actual value at the patient. Also, leakage of the connecting tube, the endotracheal tube or the respiratory mask between the ventilator and the patient can easily be detected. The potential risk of injuring the patient with the ventilation is clearly reduced. As a further advantage, damage of the lung caused by too high pressure or too high volume can be avoided.

Furthermore, according to an embodiment, a change of the lung volume that is caused by illness can be detected. The medical ventilator may consider this change of the lung volume. Furthermore, incipient spontaneous breathing and cough of the patient can be detected and also considered.

The principle according to the embodiments can help to set the ventilator to better fulfill an individual patient's demand. Also, a better respiration monitoring helps to reduce the time needed to remove the ventilator from the patient.

In the following, a further aspect of the disclosure is explained which is directed to controlling a ventilator. According to this aspect, a medical ventilator 120 (Fig. 2) is controlled based on the characterization provided by the neural network 116 as explained above. The following aspects related to ventilator control may be combined with any one or more of the embodiments explained above.

A medical ventilator (or simply "ventilator" in the following) is a machine designed to mechanically move breathable air into and out of the lungs of a vertebrate animal, e.g. a human being, to provide the mechanism of breathing for a patient who is physically unable to breathe, or breathing insufficiently. In the first case the medical ventilation is a method to replace spontaneous breathing, and in the second case it is a method to mechanically assist the spontaneous breathing.

Ventilators are chiefly used in intensive care medicine, home care, and emergency medicine (e.g., as standalone units) and in anesthesia (e.g., as a component of an anesthesia machine).

According to an embodiment, a positive pressure ventilator consists of a compressible air reservoir or turbine, air and oxygen supplies, a set of valves and tubes, and a disposable or reusable "patient circuit". The air reservoir is pneumatically compressed several times a minute to deliver room-air, or in most cases, an air/oxygen mixture to the patient. If a turbine is used, the turbine pushes air through the ventilator, with a flow valve adjusting pressure to meet patient-specific parameters. When overpressure is released, the patient will exhale passively due to the lungs' elasticity, the exhaled air being released usually through a one-way valve within the patient circuit called the patient manifold. The oxygen content of the inspired gas can be set from 21 percent (ambient air) to 100 percent (pure oxygen). Pressure and flow characteristics can be set mechanically or electronically.

According to further embodiments, ventilators may also be equipped with monitoring and alarm systems for patient-related parameters (e.g. pressure, volume, and flow) and ventilator function (e.g. air leakage, power failure, and mechanical failure), backup batteries, oxygen tanks, and remote control. The pneumatic system is nowadays often replaced by a computer-controlled turbo pump.

According to further embodiments, ventilators may be electronically controlled by an embedded system to allow exact adaptation of pressure and flow characteristics to an individual patient's needs. Fine-tuned ventilator settings also serve to make ventilation more tolerable and comfortable for the patient. As an example, in Canada, and the United States, respiratory therapists are responsible for tuning these settings while biomedical technologists are responsible for the maintenance.

According to further embodiments, the patient circuit of a ventilator may consist of a set of three durable, yet lightweight plastic tubes, separated by function (e.g. inhaled air, patient pressure, exhaled air). Determined by the type of ventilation needed, the patient-end of the circuit may be either noninvasive or invasive.

Noninvasive methods, which are adequate for patients who require a ventilator only while sleeping and resting, mainly employ a nasal mask. Invasive methods require intubation, which for long-term ventilator dependence will normally be a tracheotomy cannula, as this is much more comfortable and practical for long-term care than is larynx or nasal intubation.

Because the failure of a mechanical ventilation system may result in death, it is classified as a life-critical system, and precautions must be taken to ensure that mechanical ventilation systems are highly reliable. This also includes their power-supply provision.

According to a further aspect, as a consequence, medical ventilators are therefore carefully designed so that no single point of failure can endanger the patient. They may e.g. have manual backup mechanisms to enable hand-driven respiration in the absence of power (such as the mechanical ventilator integrated into an anesthetic machine). They may also have safety valves, which open to atmosphere in the absence of power to act as an anti-suffocation valve for the spontaneously breathing patient. Some systems may also be equipped with compressed-gas tanks, air compressors, and/or backup batteries to provide ventilation in case of power failure or defective gas supplies, and methods to operate or call for help if their mechanisms or software fail.

The main problems of conventional mechanical ventilators are the control of the configured pressure and flow characteristics (e.g. pressure, volume and flow) to guarantee an optimal maintenance of the patient. Furthermore, air leakage between the medical ventilator and the lunge must be avoided.

Some conventional ventilators are measuring the preset parameter of pressure, volume, and flow over the time. However, with these conventional systems, leakage of the connecting tube, the endotracheal tube or the respiratory mask between the ventilator and the patient are heavy to detect. Incipient spontaneous breathing and cough of the patient is also heavy to detect. Furthermore, a change of the lung volume which may be caused by illness can't be detect.

In most cases of medical ventilation using conventional ventilators, the ventilator's settings may not be optimized for the individual patient. This implies a high potential risk of injuring the patient with the ventilation. Ventilators that are not optimized to an individual patient may extend the time needed to remove the ventilator from the patient.

In view of this, some embodiments feature a control of a medical ventilator based on remote and contactless millimeter-wave radar, as explained above with reference to Figures 1 to 9B. According to an embodiment, with the remote and contactless millimeter-wave radar the respiration and the pulmonary function can be monitored. According to a further embodiment, the instantaneous measured parameter can be acknowledged to the medical ventilator for control and regulation of the configured parameters.

As the emitted radar light RLi (Fig. 1) is reflected by the body and by the changing chest features, an analysis of the reflected radar light RLr enables contactless and remote medical monitoring of the respiration and the pulmonary function. The Doppler effect shifts and broadens the frequency of the reflected radar light RLr. While the frequency shift is characteristic for the radial velocity of the chest features from the radar transmitter 101, the spectral broadening is characteristic of internal movements of the chest features, such as proper motions caused by respiration. The characteristic spectral broadening of the moving chest features is called a Doppler signal and, with the proper selection of the radar frequency, may produce an acoustic sound signal as the difference between the reflected and the emitted radar echo signal.

According to an embodiment, the configuration explained above with reference to Fig. 6A, 6B may be used for control and/or regulation of a medical ventilator 120 (Fig. 2). As with the embodiments explained above, the Doppler broadened radar echo signal received at the radar antenna, e.g. the reflected radar light RLr, may be used as an input signal, cf. reference sign S1 of Fig. 6A. In the present embodiment, the neural network 116 may be configured to characterize the patient's respiration and/or pulmonary function, and the output signal S3 (Fig. 6A) of the neural network 116 may be used to control and/or regulate the medical ventilator 120 (Fig. 2).

According to an embodiment, a table similar to that depicted by Figure 8 may be used to show a mapping of the output layer classifications of the neural network 116 (Fig. 6A) according to the patient's respiration and/or pulmonary function. In the present embodiment, said output of the neural network may control and/or regulate the medical ventilator 120.

According to an embodiment, when using the neural network 116 to control a medical ventilator 120, the neural network may be trained and/or configured such that a classification result of the neural network is determined by the activation of the nodes in the uppermost layer, i.e. the output layer L3 (Fig. 7), wherein a greatest probability is that a dedicated phase of the medical ventilation belongs to the class whose output node exhibits the highest activation. In this context, Figure 10 schematically depicts a respiration pressure curve (pressure over time) of a volume-controlled respiration with a constant flow according to an embodiment.

In the diagram of Fig. 10, the first time interval T1 represents a flow phase, the second time interval T2 represents a no-flow phase ("respiratory break"), and the third time interval T3 represents an expiration time. The combination of the first and second time interval is also denoted as inspiration time.

The pressure value P4 represents a peak pressure, the pressure value P5 represents a plateau pressure, the pressure difference P6 represents a resistance pressure, and the pressure difference P7 represents a compliance pressure.

According to an embodiment, the neural network 116 may be used to make a classification or characterization, respectively, of the different phases of the medical ventilation as exemplarily depicted by Fig. 10.

According to an embodiment, based on the aforementioned characterization by the neural network 116, a continuous mandatory ventilation by using the ventilator 120 (Fig. 2) may be provided in form of a volume-controlled respiration. According to a further embodiment, a pressure-controlled respiration may be provided based on said characterization. The ventilator control according to the embodiments addresses the various shortcomings of conventional medical ventilators explained above.

The description and drawings merely illustrate the principles of the invention. It will thus be appreciated that those skilled in the art will be able to devise various arrangements that, although not explicitly described or shown herein, embody the principles of the invention and are included within its scope. Furthermore, all examples recited herein are principally intended expressly to be only for pedagogical purposes to aid the reader in understanding the principles of the invention and the concepts contributed by the inventor(s) to furthering the art, and are to be construed as being without limitation to such specifically recited examples and conditions.

It should be appreciated by those skilled in the art that any block diagrams herein represent conceptual views of illustrative circuitry embodying the principles of the invention. Similarly, it will be appreciated that any flow charts, flow diagrams, state transition diagrams, pseudo code, and the like represent various processes which may be substantially represented in computer readable medium and so executed by a computer or processor, whether or not such computer or processor is explicitly shown.

A person of skill in the art would readily recognize that steps of various above-described methods can be performed by programmed computers. Herein, some embodiments are also intended to cover program storage devices, e.g., digital data storage media, which are machine or computer readable and encode machine-executable or computer-executable programs of instructions, wherein said instructions perform some or all of the steps of said above-described methods. The program storage devices may be, e.g., digital memories, magnetic storage media such as a magnetic disks and magnetic tapes, hard drives, or optically readable digital data storage media. The embodiments are also intended to cover computers programmed to perform said steps of the above-described methods.

The functions of the various elements shown in the FIGs., including any functional blocks labeled as "processors", may be provided through the use of dedicated hardware as well as hardware capable of executing software in association with appropriate software. When provided by a processor, the functions may be provided by a single dedicated processor, by a single shared processor, or by a plurality of individual processors, some of which may be shared. Moreover, explicit use of the term "processor" or "controller" should not be construed to refer exclusively to hardware capable of executing software, and may implicitly include, without limitation, digital signal processor (DSP) hardware, network processor, application specific integrated circuit (ASIC), field programmable gate array (FPGA), read only memory (ROM) for storing software, random access memory (RAM), and non-volatile storage. Other hardware, conventional and/or custom, may also be included. Similarly, any switches shown in the FIGS. are conceptual only. Their function may be carried out through the operation of program logic, through dedicated logic, through the interaction of program control and dedicated logic, or even manually, the particular technique being selectable by the implementer as more specifically understood from the context.

It should be appreciated by those skilled in the art that any block diagrams herein represent conceptual views of illustrative circuitry embodying the principles of the invention. Similarly, it will be appreciated that any flow charts, flow diagrams, state transition diagrams, pseudo code, and the like represent various processes which may be substantially represented in computer readable medium and so executed by a computer or processor, whether or not such computer or processor is explicitly shown.

**Claims**

1. A method of monitoring one or more of a heartbeat of a live vertebrate animal and a respiration rate of the live vertebrate animal, the method comprising: measuring (200) a frequency spectrum of radar light reflected off the live vertebrate animal; determining (210) amplitudes of a selected proper subset of the Fourier components of said frequency spectrum; and feeding (220) the amplitudes of the Fourier components of the proper subset and/or parameters derived from said Fourier components of the proper subset into a neural network (116) to make a characterization of the heartbeat and/or respiration rate of the live vertebrate animal, **characterised in that** the measured amplitudes of the Fourier components of the selected proper subset have larger variances than the measured amplitudes of remaining ones of the Fourier components.

2. Method according to claim 1, wherein cepstral coefficients associated with said proper subset are determined (212) and fed into said neural network (116).

3. Method according to claim 2, wherein a principal component analysis, PCA, is applied (214) to said cepstral coefficients, whereby principal components associated with said cepstral coefficients are obtained, and wherein said principal components are fed into said neural network (116).

4. Apparatus (100) for monitoring one or more of a heartbeat of a live vertebrate animal and a respiration rate of the live vertebrate animal, the apparatus (100) comprising: a source (102) of radar light; a light detector (104) to measure a frequency spectrum of radar light reflected off the live vertebrate animal; a digital data processor (112) being configured to receive said measured frequency spectrum from the light detector (104) and to determine amplitudes of a selected proper subset of the Fourier components of said frequency spectrum; and being configured to make a characterization of the heartbeat and/or respiration rate of the live vertebrate animal based on said Fourier components of the proper subset, **char-**

**acterised in that** the digital data processor (112) is configured such that measured amplitudes of the Fourier components of the selected proper subset have larger variances than the measured amplitudes of remaining ones of the Fourier components.

5. The apparatus (100) according to claim 4, wherein the apparatus (100) includes a neural network (116), and wherein the apparatus (100) is configured to feed the amplitudes of the Fourier components of the proper subset and/or parameters derived from said Fourier components of the proper subset into a neural network (116).

6. The apparatus (100) according to one of the claims 4 to 5, wherein the apparatus (100) is configured to determine cepstral coefficients associated with said proper subset and to feed said cepstral coefficients into said neural network (116).

7. The apparatus (100) according to claim 6, wherein said apparatus (100) is configured to apply a principal component analysis, PCA, to said cepstral coefficients, whereby principal components associated with said cepstral coefficients are obtained, and to feed said principal components into said neural network (116).

8. The apparatus according to one of the claims 4 to 7, further comprising a ventilator (120) configured to be controlled by said combination based on the characterization.

**Patentansprüche**

1. Verfahren zur Überwachung von einem oder mehreren von einem Herzschlag eines lebenden Wirbeltiers und einer Atmungsrate des lebenden Wirbeltiers, wobei das Verfahren Folgendes umfasst: Messen (200) eines Frequenzspektrums von Radarlicht, das von dem lebenden Wirbeltier weg reflektiert wird; Bestimmen (210) von Amplituden einer ausgewählten geeigneten Teilmenge der Fourier-Komponenten des Frequenzspektrums; und Eingeben (220) der Amplituden der Fourier-Komponenten der geeigneten Teilmenge und/oder Parameter, die von den Fourier-Komponenten der geeigneten Teilmenge abgeleitet wurden, in ein neuronales Netz (116), um eine Charakterisierung des Herzschlags und/oder der Atmungsrate des lebenden Wirbeltiers vorzunehmen, **dadurch gekennzeichnet, dass** die gemessenen Amplituden der Fourier-Komponenten der ausgewählten geeigneten Teilmenge größere Varianzen als die gemessenen Amplituden von übrigen von den Fourier-Komponenten aufweisen.

2. Verfahren nach Anspruch 1, wobei Cepstrum-Koeffizienten, die der geeigneten Teilmenge zugehörig sind, bestimmt (212) und in das neuronale Netz (116) eingegeben werden.

3. Verfahren nach Anspruch 2, wobei eine Hauptkomponentenanalyse, PCA, auf die Cepstrum-Koeffizienten angewandt wird (214), wodurch Hauptkomponenten, die den Cepstrum-Koeffizienten zugehörig sind, erhalten werden, und wobei die Hauptkomponenten in das neuronale Netz (116) eingegeben werden.

4. Vorrichtung (100) zur Überwachung von einem oder mehreren von einem Herzschlag eines lebenden Wirbeltiers und einer Atmungsrate des lebenden Wirbeltiers, wobei die Vorrichtung (100) Folgendes umfasst: eine Quelle (102) von Radarlicht; einen Lichtdetektor (104) zum Messen eines Frequenzspektrums von Radarlicht, das von dem lebenden Wirbeltier weg reflektiert wird; einen digitalen Datenprozessor (112), der dazu ausgestaltet ist, das gemessene Frequenzspektrum von dem Lichtdetektor (104) zu empfangen und Amplituden einer ausgewählten geeigneten Teilmenge der Fourier-Komponenten des Frequenzspektrums zu bestimmen; und dazu ausgestaltet ist, eine Charakterisierung des Herzschlags und/oder der Atmungsrate des lebenden Wirbeltiers basierend auf den Fourier-Komponenten der geeigneten Teilmenge vorzunehmen, **dadurch gekennzeichnet, dass** der digitale Datenprozessor (112) derart ausgestaltet ist, dass gemessene Amplituden der Fourier-Komponenten der ausgewählten geeigneten Teilmenge größere Varianzen als die gemessenen Amplituden von übrigen von den Fourier-Komponenten aufweisen.

5. Vorrichtung (100) nach Anspruch 4, wobei die Vorrichtung (100) ein neuronales Netz (116) umfasst und wobei die Vorrichtung (100) dazu ausgestaltet ist, die Amplituden der Fourier-Komponenten der geeigneten Teilmenge und/oder Parameter, die von den Fourier-Komponenten der geeigneten Teilmenge abgeleitet werden, in ein neuronales Netz (116) einzugeben.

6. Vorrichtung (100) nach einem der Ansprüche 4 bis 5, wobei die Vorrichtung (100) dazu ausgestaltet ist, Cepstrum-Koeffizienten, die der geeigneten Teilmenge zugehörig sind, zu bestimmen und die Cepstrum-Koeffizienten in das neuronale Netz (116) einzugeben.

7. Vorrichtung (100) nach Anspruch 6, wobei die Vorrichtung (100) dazu ausgestaltet ist, eine Hauptkomponentenanalyse, PCA, auf die Cepstrum-Koeffizienten anzuwenden, wodurch Hauptkomponenten, die den Cepstrum-Koeffizienten zugehörig sind, erhalten werden, und die Hauptkomponenten in das neuronale Netz (116) einzugeben.

8. Vorrichtung nach einem der Ansprüche 4 bis 7, die ferner einen Ventilator (120) umfasst, der dazu ausgestaltet ist, durch die Kombination basierend auf der Charakterisierung gesteuert zu werden.

**Revendications**

1. Procédé de surveillance d'un ou plusieurs parmi les battements de cœur d'un animal vertébré vivant et le rythme respiratoire de l'animal vertébré vivant, le procédé comprenant : la mesure (200) du spectre de fréquences d'une lumière radar réfléchie par l'animal vertébré vivant ; la détermination (210) d'amplitudes d'un sous-ensemble approprié sélectionné des composantes de Fourier dudit spectre de fréquences ; et l'introduction (220) des amplitudes des composantes de Fourier du sous-ensemble approprié et/ou de paramètres dérivés desdites composantes de Fourier du sous-ensemble approprié dans un réseau neural (116) pour réaliser une caractérisation des battements de cœur et/ou du rythme respiratoire de l'animal vertébré vivant, **caractérisé en ce que** les amplitudes mesurées des composantes de Fourier du sous-ensemble approprié sélectionné ont des variances supérieures à celles des amplitudes mesurées de celles restantes des composantes de Fourier.

2. Procédé selon la revendication 1, dans lequel les coefficients cepstraux associés audit sous-ensemble approprié sont déterminés (212) et introduits dans ledit réseau neural (116) .

3. Procédé selon la revendication 2, dans lequel une analyse de composantes principales, PCA, est appliquée (214) auxdits coefficients cepstraux, moyennant quoi les composantes principales associées auxdits coefficients cepstraux sont obtenues, et dans lequel lesdites composantes principales sont introduites dans ledit réseau neural (116).

4. Dispositif (100) pour surveiller un ou plusieurs parmi les battements de cœur d'un animal vertébré vivant et le rythme respiratoire de l'animal vertébré vivant, le dispositif (100) comprenant : une source (102) de lumière radar ; un détecteur de lumière (104) pour mesurer le spectre de fréquences de la lumière radar réfléchie par l'animal vertébré vivant ; un processeur de données numériques (112) configuré pour recevoir ledit spectre de fréquences mesurées depuis le détecteur de lumière (104) et pour déterminer les amplitudes d'un sous-ensemble approprié sélectionné des composantes de Fourier dudit spectre de fréquences ; et configuré pour réaliser une caractérisation des battements de cœur et/ou du rythme respiratoire de l'animal vertébré vivant sur la base desdites composantes de Fourier du sous-ensemble approprié, **caractérisé en ce que** le processeur de données numériques (112) est configuré de façon que les amplitudes mesurées des composantes de Fourier du sous-ensemble approprié sélectionné aient des variances supérieures à celles des amplitudes mesurées de celles restantes des composantes de Fourier.

5. Dispositif (100) selon la revendication 4, lequel dispositif (100) comprend un réseau neural (116), et lequel dispositif (100) est configuré pour introduire les amplitudes des composantes de Fourier du sous-ensemble approprié et/ou de paramètres dérivés desdites composantes de Fourier du sous-ensemble approprié dans un réseau neural (116).

6. Dispositif (100) selon l'une quelconque des revendications 4 et 5, lequel dispositif (100) est configuré pour déterminer des coefficients cepstraux associés audit sous-ensemble approprié et à introduire lesdits coefficients cepstraux dans ledit réseau neural (116).

7. Dispositif (100) selon la revendication 6, lequel dispositif (100) est configuré pour appliquer une analyse de composantes principales, PCA, auxdits coefficients cepstraux, moyennant quoi les composantes principales associées auxdits coefficients cepstraux sont obtenues, et pour introduire lesdites composantes principales dans ledit réseau neural (116) .

8. Dispositif selon l'une quelconque des revendications 4 à 7, comprenant en outre un ventilateur (120) configuré pour être commandé par ladite combinaison sur la base de la caractérisation.

## Fig. 1

10

101

12a

P

RLr

RLi

12

## Fig. 2

100

112    114    102    101    104

110

116

120

A

## Fig. 3A

## Fig. 3B

## Fig. 4

# Fig. 5

# Fig. 6A

# Fig. 6B

# Fig. 7

# Fig. 8

| Output#1 | 1 |
|---|---|
| Output#2 | 2 |
| Output#3 | 3 |
| . | . |
| . | . |
| Output#m-2 | m-2 |
| Output#m-1 | m-1 |
| Output#m | m |

Fig. 9A

## Fig. 9B

## Fig. 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 102009033829 A1
- US 2016089052 A1